# EUROPEAN PATENT SPECIFICATION

(11) **EP 3 860 338 B1**
(45) Date of publication and mention of the grant of the patent: **22.01.2025**
(21) Application number: 19778784.9
(22) Date of filing: 07.10.2019
(51) Int. Cl.: A01K 67/033

(54) **MITE COMPOSITION AND METHOD FOR REARING MITES**
MILBEN ZUSAMMENSETZUNG UND VERFAHREN ZUM ZÜCHTEN VON MILBEN
COMPOSITION DE MITES ET MÉTHODE D'ÉLEVAGE DE MITES

(30) Priority: 05.10.2018 BE 201805688
(43) Date of publication of application: 11.08.2021
(73) Proprietor: Biobest Group N.V., 2260 Westerlo (BE)
(72) Inventor: VANGANSBEKE, Dominiek, 2260 Westerlo (BE); DUARTE, Marcus Vinicius Alfenas, 2260 Westerlo (BE); GUILBAUD, Manon Hélène Lionella, 2260 Westerlo (BE); BENAVENTE MARTINEZ, Alfredo, 2260 Westerlo (BE); PEKAS, Apostolos, 2260 Westerlo (BE); BOLCKMANS, Karel Jozef Florent, 2260 Westerlo (BE); WÄCKERS, Felix Leopold, 2260 Westerlo (BE)
(74) Representative: Gevers Patents
(86) International application number: PCT/EP2019/077115
(87) International publication number: WO 2020/070334

(56) References cited:
- WO-A1-2006/057552
- WO-A2-2008/104807
- MCMURTHY J A ET AL: "NUTRITIONAL ECOLOGY OF PHYTOSEIID MITES", NUTRITIONAL ECOLOGY OF INSECTS, MITES, SPIDERS AND RELATEDINVERTEBRATES, WILEY, NEW YORK, NY, US, 1 January 1987 (1987-01-01), pages 609 - 644, XP008068054

## Description

### Field of the invention

The present invention relates to methods for the mass rearing of predatory mites, mite compositions and the use thereof for biological control. In particular, the present invention relates to the use of fungus-feeding tarsonemid prey mites for the mass rearing of predatory mites and for controlling a pest in a crop.

### Background of the invention

Predatory mites are traditionally used in the broad field of agricultural pest management. A wide range of predatory mite species have been suggested or commercialized for the biological control of phytophagous pest mites and insect pests such as whiteflies and thrips.

In recent years, the major arthropod pest species involved have generally been controlled by chemical agents i.e. pesticides. Many newer pesticides made available in the past decade are more selective and less hazardous than most of the older compounds. However, it is becoming increasingly clear that the strategy of unilateral reliance on chemical control will not be the solution to the problem. In this regard, there are several major problems attendant to chemical control i.e. the development of resistance to chemicals in target pest species; the dwindling supply of useful, registered insecticides and acaricides; the deposit of some undesirable residues; the damaging (or detrimental) effect of these chemicals on non-target species resulting in secondary pest outbreaks (contaminate soil and water affect wildlife, aquatic life, and other non-target organisms and interfere with beneficial organisms such as pollinating insects and the natural enemies of pests); and the phytotoxic reactions by treated plants. For these reasons, many farmers and gardeners are exploring and adopting methods that reduce pesticide use.

Biological control represents one alternative to the use of chemical agents. Biological control is the intentional manipulation of populations of living beneficial organisms (natural enemies) in order to limit population of pests. Virtually all pests have natural enemies and appropriate management of natural enemies can effectively control many pests. Biological control can be effective, economical and safe. The intent of biological control is not to eradicate pests, but to keep them at tolerable levels at which they cause no appreciable damage.

Natural enemies of mites include predators, parasitic insects, nematodes, and pathogens. Many publications already demonstrate the use of a predatory mite population in order to control pests. For example, currently, primarily phytoseiid predatory mites are employed to combat pests such as phytophagous mites, thrips and whiteflies. As one example, *Neoseiulus cucumeris* (Oudemans) is commercially used for the control of thrips larvae and spider mites. In addition, other predatory mite species selected from mesostigmatid and prostigmatid predatory species receive attention in biological pest control and some have entered market.

It is therefore an interest of developing effective mass rearing systems to produce predatory mite populations on a commercially relevant scale for an acceptable price. In the present commercial rearing systems populations of the predatory mites are reared on life prey in a culture maintained on a carrier. For example, WO2006071107 and WO2013103295 disclose a mite composition comprising a population of individuals of a predatory mite species, a prey mite population as a food source for the predatory mite individuals and a carrier. The composition according to this prior art document is suitable for rearing a mite species and for the biological control of pests. The most commonly used prey mites in the mass rearing methods of the prior art are Astigmatid mites, such as *Tyrophagus putrescentiae* (Schrank), *Thyreophagus entomophagous* (Laboulbéne & Robin) and *Carpoglyphus lactis* L.. Up to date, all prey mite species being used in commercial mass-rearing of predatory mites belong to the order Astigmata. For example, WO 2006/056552, WO 2008/015393, WO 2008/104807 and WO 2007/075081 demonstrate the potential of Astigmatids to be used as prey mites in mass-rearing of predatory mites.

Pest mites are sometimes used to study the interaction between predatory mites and the pest mites. For example, *Polyphagotarsonemus latus* (Rodriguez Morell et al., 2010, International Journal of Acarology 36 (5): 371-376) and *Phytonemus pallidus* (Tuovinen & Lindqvist , 2010, Biological Control 54 (2): 119-125) mites from the family of Tarsonemidae were grown on lab-scale on living plant material and were used to study attacks by predatory mites. XP008065054 (NUTRITIONAL ECOLOGY OF PHYTOSEIID MITES, J.A. McMurtry, J.G. Rodriguez) suggests to investigate the importance of Fungus-feeding tarsonemids as alternate prey for phytoseiids.

However, mite compositions with such prey mite populations are not a realistic solution for commercial culturing since these are known as pests that should not be distributed.

Mass rearing systems for predatory mites depend heavily on the availability of suitable prey for the predators. In view of their role in rearing of predatory mites, the commercial relevance of rearing prey mites is increasing.

In view of the above there is a continuing need to obtain improved (more efficient) mite compositions for mass rearing and large-scale production of predatory mite populations, for commercial distribution of larger volumes of mite compositions comprising such and for better pest control and agricultural management.

In addition, commercial mite rearing requires rearing conditions at a substantial temperature (generally between 20 and 30 °C) and high humidity (generally between 80 and 95% relative humidity). In these conditions, problems of fungal contamination easily occur. Therefore, there is also need for improved mite rearing methods that lower the risk of fungal contamination.

### Summary of the invention

The inventors have surprisingly found that the use of tarsonemids as prey mites overcome the problems of the prior art.

Therefore the present invention provides a mite composition comprising
- a predatory mite population,
- a prey mite population, and
- a carrier for individuals of said populations,

wherein said prey mite population comprises fungus-feeding mites from the family of the Tarsonemidae,
wherein said mite composition comprises at least 1,000 mites of the prey mite population per gram of the composition, and
wherein said carrier is a solid material which is suitable to provide a carrier surface to the individuals of both the predatory and the prey mite populations,
wherein said mite composition does not comprise a living mite species selected from the group consisting of *Polyphagotarsonemus latus* and *Phytonemus pallidus.*

### Brief description of the drawings

Fig. 1: Number of eggs deposited by a female *A. swirskii* for 3 consecutive days when fed *T. fusarii* (TF), C. lactis (CL) or *T. entomophagus* (TE) at 22 ± 1°C and 85 ± 5% RH. Different letters above the bars denote significant differences among treatments (contrasts after GLM, p<0.05).
Fig. 2: Capture success rate of *A. swirskii* females on eggs, larvae and adults of *T. fusarii* (TF), *C. lactis* (CL) and *T. entomophagus* (TE). Different letters indicate significant differences between the prey mite species per developmental stage (contrasts after GLM, p<0.05).
Fig. 3: Number of eggs deposited by female *H. anconai* for 3 consecutive days when fed *T. fusarii* as compared to a control consisting only of a tomato leaf arena (22 ± 1°C and 85 ± 5% RH).
Fig. 4: Number of eggs deposited by female *P. ubiquitus* for 3 consecutive days when fed *T. fusarii* as compared to *C. lactis* and a control treatment without prey mites (22 ± 1°C and 85 ± 5% RH).
Fig. 5: Number of eggs deposited by female *A. swirskii* for 2 consecutive days when fed either *T. fusarii* or *T. confusus* (22 ± 1°C and 85 ± 5% RH).
Fig. 6: Number of eggs deposited by female *A. swirskii* for 2 consecutive days when fed *Fungitarsonemus sp.* as compared to a control consisting only of an ivy leaf arena (22 ± 1°C and 85 ± 5% RH).

### Detailed description of the invention

As described herein before, the present invention provides a mite composition comprising
- a predatory mite population,
- a prey mite population, and
- a carrier for individuals of said populations,

wherein said prey mite population comprises fungus-feeding mites from the family of the Tarsonemidae,
wherein said mite composition comprises at least 1,000 mites of the prey mite population per gram of the composition, and
wherein said carrier is a solid material which is suitable to provide a carrier surface to the individuals of both the predatory and the prey mite populations,
wherein said mite composition does not comprise a living mite species selected from the group consisting of *Polyphagotarsonemus latus* and *Phytonemus pallidus.*

By the term « predatory mite population », is meant in the sense of the present invention, a population of beneficial mites that feeds on a prey mite population.

By the term « prey mite population », is meant in the sense of the present invention, a population of mites that at least partially consumed by a predatory mite population.

By the term « carrier », is meant in the sense of the present invention, any solid material which is suitable to provide a carrier surface to the individuals of both the predatory and the prey mite populations. The carrier will usually act as a three-dimensional matrix wherein the prey mite population and predatory mite population can move around, hide, develop and prey or be preyed upon.

Inventors surprisingly observed that a mite composition according to the invention shows better results in terms of efficiency. It was observed that the mites from the family of Tarsonemidae of the prey mite population are more easily killed and more readily eaten by the predatory mite population than the conventionally used astigmatid prey mites. An additional advantage is that all life stages of the tarsonemid prey mites are being killed readily, whereas larger astigmatid life stages, such as adults, are being killed at a much lower rate. In addition, it was observed that at even at high densities, the prey mite population disturbs the predatory mite population less than the astigmatid prey mites used for mass rearing mites. Although not wishing to be bound by theory, it appears that the tarsonemid mites might not produce repellent volatiles or produce them to a much lower degree, compared to astigmatid mites. This may cause the tarsonemids to have a lower disturbing effect on the predatory mite population. Accordingly, individuals from the predatory mite population may grow faster and therefore mature more quickly which allows for a higher rate of reproduction. Such a higher reproductive rate results in a faster increase in the number of individuals in the predatory mite population. This advantage is beneficial for the rearing of predatory mites and the production of compositions comprising such, as well as for the control of a pest in a crop. It is to be noted that the prey mite population used in the invention may also comprise (or consist of) a population of dead prey mites. The observation that the prey mites of the invention are more readily eaten than the prey mites of the prior art, such as astigmatid mites, also applies to dead tarsonemid mites in comparison to dead astigmatid mites. The use of a prey mite population comprising dead prey mites provides the benefit that predatory mites require less energy for killing and consuming the prey mites.

Secondly, it was observed that the appearance of mold is prevented with the mite composition according to the invention. Indeed, it was observed that mites from the family of Tarsonemidae feed on fungi. Surprisingly, the tarsonemid prey mites not only reduce fungal growth, but are able to completely suppress it to a non-visible degree in commercially relevant rearing conditions. Indeed, as discussed herein before, for the rearing of predatory mites for the purpose of production and distribution, it is essential to maintain strict conditions of temperature and humidity. A hot and humid environment is ideal for the rearing of predatory mites. Unfortunately, such specific conditions favor fungal growth, which severely limit the ability to rear mites and to produce commercially acceptable predatory mite compositions. The composition according to the invention, contrary to mite compositions from the state of the art, allows mass rearing and thus commercial distribution of larger volumes, both leading to cost reduction. Therefore, preferably, the prey mite population comprises living mites from the family of the Tarsonemidae. The use of living mites according to the invention provides a number of benefits over the use of dead mites, such as the suppression of fungal contamination.

In a particular embodiment, the prey mite population as used in the invention comprises (a) a population of dead prey mites, and (b) a population of living mites from the family of the Tarsonemidae. As the use of dead prey mites often cause fungal contamination of rearing cultures, the addition of living prey mites according to the invention suppresses this problem. In a more particular embodiment, the prey mite population as used in the invention comprises (a) a population of dead prey mites from the family of the Tarsonemidae, and (b) a population of living mites from the family of the Tarsonemidae.

By the term « mold », is meant in the sense of the present invention, a fungus that grows in the form of multicellular filaments called hyphae. Molds are a large and taxonomically diverse number of fungal species causing biodegradation of natural materials. Molds do not form a specific taxonomic or phylogenetic grouping but can be found in the divisions *Zygomycota* and *Ascomycota.* Common genera of molds include *Acremonium, Alternaria, Aspergillus, Cladosporium, Fusarium, Mucor, Penicillium, Rhizopus, Stachybotrys, Trichoderma* and *Trichophyton.* In the sense of the present invention, the term « mold » also comprises mildew, a closely related counterpart of mold, which is either species of fungus in the order *Erysiphales*, or fungus-like organisms in the family *Peronosporaceae.* Thirdly, the tarsonemid mites according to the invention do not cause significant plant damage, contrary to *Polyphagotarsonemus latus* and *Phytonemus pallidus* mites which are considered as sub-major pests at low elevations in summer months. Actually, mites from the species *P. latus* and *P. pallidus* attack many fruit and vegetable crops in temperate and subtropical areas. Accordingly, the composition according to the invention, contrary to mite compositions comprising *Polyphagotarsonemus latus* and *Phytonemus pallidus* mites allow both mass rearing and pest control.

Advantageously, the composition according to the invention may further comprise a fungus. Better efficiency results were observed when fungi are added to the composition according to the invention. In fact, the prey mite population feeds on fungi. Thus, the active addition of fungi in the composition according to the invention makes it possible to improve the growth and therefore to accelerate the reproduction maturity of Tarsonemidae, which will lead to a faster increase of Tarsonemidae individuals of the prey mite population. In addition, it has been observed that the active addition of beneficial fungi stabilizes the rearing cultures and further suppresses the growth of unwanted fungal contaminations. Accordingly, the active addition of fungi in the composition according to the invention will indirectly enhance the rearing of predator mite population, their large-scale production and the distribution of the composition according to the invention at larger volumes but will also show better effects on pest control on crops. In a preferred embodiment, the fungus belongs to the Hypocreaceae, in particular, the fungi are selected from *Gliocladium* and *Trichoderma.*

"Tarsonemid" as used herein refers to a mite species of the family of Tarsonemidae, both terms are used interchangeably herein. The tarsonemidae are a family of mites, also called thread footed mites or white mites. In a particular embodiment, the tarsonemid mites belong to the subfamily of Tarsoneminae, more in particular to the tribe of Hemitarsonemini, Steneotarsonemini, Tarsonemini, or Pseudacarapini.

In a preferred embodiment, the tarsonemid mites are selected from the group consisting of the genera *Tarsonemus* (e.g. *Tarsonemus granarius, Tarsonemus floricolus*, *Tarsonemus myceliophagus*, *Tarsonemus subcorticalis*, Tarsonemus minimax, *Tarsonemus fusarii*), *Fungitarsonemus* (e.g. *Fungitarsonemus peregrinus, Fungitarsonemus pulvirosus*), *Heterotarsonemus* (e.g. *Heterotarsonemus lindquisti*, *Heterotarsonemus coleopterorum*), *Daidalotarsonemus* (e.g. *Daidalotarsonemus vandevriei*, *Diadalotarsonemus tesselatus*), *Neotarsonemoides* (e.g. *Neotarsonemoides denigrates*), and *Pseudacarapis* (e.g. *Pseudacarapis indoapis*). In a more preferred embodiment, the prey mites of the invention are selected from the genus *Tarsonemus*; more in particular from the group consisting of *Tarsonemus granarius*, *Tarsonemus floricolus*, *Tarsonemus myceliophagus*, *Tarsonemus subcorticalis*, *Tarsonemus minimax*, and *Tarsonemus fusarii.*

Preferably, the tarsonemid mites according to the invention do not cause significant plant damage. The skilled person is well aware how to select tarsonemid mites that do not cause significant plant damage. In addition, the absence of causing significant plant damage is easily determined, e.g. by incubating a plant with the tarsonemid of choice and determining if the plant is damaged after a few days. In a particular embodiment, the prey mites according to the invention include mites from the family of the Tarsonemidae, wherein said mites from the family of the Tarsonemidae are not selected from the group comprising *Polyphagotarsonemus latus* and *Phytonemus pallidus.* As will be understood from the context herein, the tarsonemid mites according to the invention are fungus-feeding tarsonemid mites.

Advantageously, the composition according to the invention comprises a food source for the predatory and/or prey mite populations. In a particular embodiment, the composition of the invention comprises a food source for said prey mite population. As also described elsewhere, the food source for said prey mite population may comprise a fungus. In another embodiment, the food source for said prey mite population comprises non-photosynthetic plant material. In a further embodiment, the food source comprises wheat germ or pollen. As is known to the skilled person, a carrier can be selected that acts both as a carrier and as a food source, for example wheat germ.

In a particular embodiment, the composition of the invention further comprises a supplemental food source for the predatory mite population. A supplemental food source as used herein refers to a food source that is present in addition to the prey mite population. Supplemental food sources are sometimes used in predatory mite rearing as a food source that is more cost-effective than prey mites. It is often observed that several predatory mites can use supplemental foods for obtaining additional energy, but also require the presence of prey mites for survival, development and reproduction. A major drawback of the use of a supplemental food source is that it is prone to fungal contamination. By combining a supplemental food source with the prey mites of the invention, this drawback is suppressed. Examples of preferred supplemental foods are natural food sources (such as pollen or dead mites) and artificial foods (Wäckers et al. (2005) Plant-provided food for carnivorous insects: a protective mutualism and its applications 356p; Morales-Ramos et al. (2014) Mass production of beneficial organisms 742p; Cohen (2015) Insect diets. Science and Technology, Second edition, 473p) Supplemental food sources for the rearing of predatory mites are well-known to the skilled person.

As mentioned herein before, the carrier can be any solid material which is suitable to provide a carrier surface to the individuals. In a preferred embodiment, the carrier is a mixture of solid carrier elements. Preferably, the carrier is a substantially homogeneous mixture of solid carrier elements. While the solid carrier elements may have a variety of sizes, a substantially homogeneous mixture refers to the variety of sizes being substantially homogeneously distributed amongst the carrier.

The average longest axis of the solid carrier elements is typically between 1.0 and 20.0 mm, more in particular between 2.0 and 12.0 mm, preferably between 3.0 and 9.0 mm. In another particular embodiment, the carrier is a mixture of solid carrier elements with a similar size. In a further embodiment, 90% of the solid carrier elements have a longest axis in the range of 0.1 times to 10.0 times the average longest axis of the mixture of solid carrier elements, in particular in the range of 0.2 and 5.0 times, more in particular in the range of 0.5 and 2.0 times. As will be understood from the disclosures herein, two or more different types of carrier elements may be used and mixed. In such case, the size distribution refers to the size distribution per type of carrier elements. Preferably the carrier provides a porous medium, which allows exchanges of metabolic gases and heat produced by the mite populations. Examples of suitable carriers are non-photosynthetic plant material, such as (wheat) bran, buckwheat husks, rice husks, saw dust, corn cob grits, etcetera, or inorganic material, such as vermiculite. Advantageously, said carrier comprises grains of a grass species or any part thereof, such as germ or bran. Preferably, said carrier does not comprise living, green plant material, such as leaves or stems of plants. In a particular embodiment, the carrier comprises non-photosynthetic plant material. Particularly preferred is a carrier selected from vermiculite, wheat bran, millet chaff, rice husks and buckwheat husks.

Preferably, said carrier comprises carrier elements with an average longest axis of between 1.0 and 20.0 mm, in particular between 3.0 and 9.0 mm. The carrier material may provide shelter for the prey mites and predatory mites, reducing stress conditions and cannibalism. During mass rearing, the carrier allows for the generation of a three-dimensional rearing matrix and may provide a food source for the prey mites. During storage and distribution for biological control, the carrier acts as a bulking agent and allows for a more homogeneous distribution of the predatory mites in the crops.

Advantageously, the number of individuals of the predatory mite population relative to the number of individuals of the prey mite population is from about 1:1 to 1:500, such as about 1:50 to 1:200, and preferably between 1:75 and 1:125.

High densities of prey mites and predatory mites can be reached thanks to the present invention. The mite compositions of the present invention comprise at least 1,000 mites of the prey mite population per gram of the composition, in particular at least 2,000 prey mites, more in particular at least 3,000 prey mites. In a further embodiment, at least 5,000 prey mites, at least 10,000 prey mites, at least 20,000 prey mites, at least 30,000 prey mites. In a preferred embodiment, the mite composition comprises at least 50,000 mites of the prey mite population per gram of the composition.

In another particular embodiment, the mite compositions of the present invention comprise at least 20 mites of the predatory mite population per gram of the composition, in particular at least 30 predatory mites, more in particular at least 50 predatory mites. In a further embodiment, at least 100 predatory mites, at least 150 predatory mites, at least 200 predatory mites, at least 300 predatory mites. In a preferred embodiment, the mite composition comprises at least 500 mites of the predatory mite population per gram of the composition.

Mite numbers are mentioned herein refer to the total number of all mite development stages, thus including eggs, larvae, nymphs and adults, unless the context clearly dictates otherwise. As will be understood from the disclosures herein, the prey mites in the compositions of the invention are not required to be living prey mites.

In a particular embodiment, the mite compositions of the present invention comprise at least 1,000 non-egg mites of the prey mite population per gram of the composition, in particular at least 2,000 non-egg prey mites, more in particular at least 3,000 non-egg prey mites. In a further embodiment, at least 5,000 prey non-egg mites, at least 10,000 prey non-egg mites, at least 20,000 prey non-egg mites, at least 30,000 prey non-egg mites. In a preferred embodiment, the mite composition comprises at least 50,000 non-egg mites of the prey mite population per gram of the composition.

In another particular embodiment, the mite compositions of the present invention comprise at least 20 non-egg mites of the predatory mite population per gram of the composition, in particular at least 30 non-egg predatory mites, more in particular at least non-egg 50 predatory mites. In a further embodiment, at least 100 non-egg predatory mites, at least 150 non-egg predatory mites, at least 200 non-egg predatory mites, at least 300 non-egg predatory mites. In a preferred embodiment, the mite composition comprises at least 500 non-egg mites of the predatory mite population per gram of the composition.

In another particular embodiment, the mite compositions of the present invention comprise at least 20 adult mites of the predatory mite population per gram of the composition, in particular at least 30 adult predatory mites, more in particular at least 50 adult predatory mites. In a further embodiment, at least 100 adult predatory mites, at least 150 adult predatory mites, at least 200 adult predatory mites, at least 300 adult predatory mites. In a preferred embodiment, the mite composition comprises at least 500 adult mites of the predatory mite population per gram of the composition. In further embodiment, the mite compositions of the present invention comprise at least 20 living adult mites of the predatory mite population per gram of the composition, in particular at least 30 living adult predatory mites, more in particular at least 50 living adult predatory mites. In a further embodiment, at least 100 living adult predatory mites, at least 150 living adult predatory mites, at least 200 living adult predatory mites, at least 300 living adult predatory mites. In a preferred embodiment, the mite composition comprises at least 500 living adult mites of the predatory mite population per gram of the composition.

Evidently, the above ranges of prey mite and predatory mite numbers can be combined to arrive to particular and preferred embodiments of the invention. It thus follows that the present invention provides, for example, mite compositions comprising at least 1,000 mites of the prey mite population and at least 20 mites of the predatory mite population per gram of the composition. As another example, at least 5,000 mites of the prey mite population and at least 100 mites of the predatory mite population. As another example, at least 50,000 mites of the prey mite population and at least 500 mites of the predatory mite population.

In a preferred embodiment, said predatory mites are mesostigmatid or prostigmatid mite species. In a further embodiment, said predatory mites are selected from:
- Mesostigmatid predatory mite species such as:
   i) Phytoseiidae such as from:
      a)- the subfamily of the Amblyseiinae, such as from the genus *Amblyseius*, e.g. *Amblyseius andersoni*, *Amblyseius aerialis*, *Amblyseius swirskii*, *Amblyseius herbicolus* or *Amblyseius largoensis*, from the genus *Euseius* e.g. *Euseius finlandicus*, *Euseius hibisci*, *Euseius ovalis*, *Euseius victoriensis*, *Euseius stipulatus*, *Euseius scutalis*, *Euseius tularensis*, *Euseius addoensis*, *Euseius concordis*, *Euseius ho* or *Euseius citri*, from the genus *Neoseiulus* e.g. *Neoseiulus barkeri, Neoseiulus californicus*, *Neoseiulus cucumeris*, *Neoseiulus longispinosus*, *Neoseiulus womersleyi*, *Neoseiulus idaeus*, *Neoseiulus anonymus*, *Neoseiulus paspalivorus*, *Neoseiulus reductus* or *Neoseiulus fallacis*, from the genus *Amblydromalus* e.g. *Amblydromalus limonicus* from the genus *Typhlodromalus* e.g. *Typhlodromalus aripo*, *Typhlodromalus laila* or *Typhlodromalus peregrinus* from the genus Typhlodromips e.g. *Typhlodromips montdorensis*, from the genus *Phytoseiulus*, e.g. *Phytoseiulus persimilis*, *Phytoseiulus macropilis*, *Phytoseiulus longipes*, *Phytoseiulus fragariae*;
      b) the subfamily of the Typhlodrominae, such as from the genus *Galendromus* e.g. *Galendromus occidentalism* from the genus *Typhlodromus* e.g. *Typhlodromus pyri*, *Typhlodromus doreenae*, *Typhlodromus rhenanus* or *Typhlodromus athiasae*;
      c) the subfamily of the Phytoseiinae, such as from the genus Phytoseius e.g. Phytoseius macropilis, Phytososeius finitimus or Phytoseius plumifer.
   ii) Ascidae such as from the genus *Proctolaelaps,* such as *Proctolaelaps pygmaeus* (Muller); from the genus *Blattisocius* e.g. *Blattisocius tarsalis* (Berlese), *Blattisocius keegani* (Fox); from the genus *Lasioseius* e.g. *Lasioseius fimetorum* Karg, *Lasioseius floridensis* Berlese, *Lasioseius bispinosus* Evans, *Lasioseius dentatus* Fox, *Lasioseius scapulatus* (Kenett), *Lasioseius athiasae* Nawar & Nasr; from the genus *Arctoseius* e.g. *Arctoseius semiscissus* (Berlese); from the genus Protogamasellus e.g. *Protogamasellus dioscorus* Manson;
   iii) Laelapidae such as from the genus *Stratiolaelaps* e.g. *Stratiolaelaps scimitus* (Womersley) (also placed in the genus Hypoaspis) ; *Gaeolaelaps* e.g. *Gaeolaelaps aculeifer* (Canestrini) (also placed in the genus Hypoaspis); Androlaelaps e.g. *Androlaelaps casalis* (Berlese);
   iv) Macrochelidae such as from the genus *Macrocheles* e.g. *Macrocheles robustulus* (Berlese), *Macrocheles muscaedomesticae* (Scopoli), *Macrocheles matrius* (Hull);
   v) Parasitidae such as from the genus *Pergamasus*; e.g. *Pergamasus quisquiliarum* Canestrini; *Parasitus* e.g. *Parasitusfimetorum* (Berlese), *Parasitus bituberosus* Karg; Parasitellus e.g. *Parasitellus fucorum* (De Geer);
   vi) Digamasellidae such as from the genus *Digamasellus*; e.g. *Digamasellus quadrisetus* or *Digamasellus punctum*; from the genus *Dendrolaelaps*; e.g. *Dendrolaelaps neodisetus*; and
- prostigmatid mite species such as from:
   i) Tydeidae such as from the genus *Homeopronematus* e.g. *Homeopronematus anconai* (Baker); from the genus *Tydeus* e.g. *Tydeus lambi* (Baker), *Tydeus caudatus* (Duges); from the genus *Pronematus* e.g. *Pronematus ubiquitus* (McGregor);
   ii) Cheyletidae such as from the genus *Cheyletus* e.g. *Cheyletus eruditus* (Schrank), *Cheyletus malaccensis* Oudemans;
   iii) Cunaxidae such as from the genus *Coleoscirus* e.g. *Coleoscirus simplex* (Ewing), from the genus *Cunaxa* e.g. *Cunaxa setirostris* (Rermann);
   iv) Erythraeidae such as from the genus *Balaustium* e.g. *Balaustium putmani* Smiley, *Balaustium medicagoense* Meyer & Ryke , *Balaustium murorum* (Hermann);
   v) Stigmaeidae such as from the genus *Agistemus* e.g. *Agistemus exsertus* Gonzalez; such as from the genus *Zetzellia* e.g. *Zetzellia mali* (Ewing); and
   vi) Tarsonemidae such as from the genus *Acaronemus*, e.g. *Acaronemus destructor* (Smiley and Landwehr); such as from the genus Dendroptus near suski Sharonov and Livshits; such as *Lupotarsonemus floridanus* Attiah.

In a preferred embodiment, the predatory mites belong to the Phytoseiidae. In a particular embodiment, the predatory mites are selected from Amblyseiinae, *Transeius*, *Neoseiulus* and *Amblydromalus*; more in particular the predatory mites are selected from the group consisting of *Amblyseius swirskii*, *Transeius montdorensis*, *Neoseiulus californicus* and *Amblydromalus limonicus.*

In another embodiment, the predatory mites are prostigmatid mites, in particular Tydeidae. In a further embodiment, the predatory mites are from the genus *Homeopronematus* or *Pronematus*, preferably *Homeopronematus anconai* or *Pronematus ubiquitus.*

The present invention also provides a commercial packaging for storing and distributing the composition of the invention. Therefore, the present invention further pertains to a container comprising a composition according to the invention. In particular, the container has an internal volume of between 0.2 I and 3 I, preferably between 0.5 I and 2 I. According to a preferred embodiment the container preferably comprising an exit for at least one motile life stage of the mite, more preferably an exit suitable for providing a sustained release of said at least one motile life stage. In a particular embodiment, the container (e.g. a bottle or a sachet) has at least one exit with a removable seal. In particular, seal as used herein refers to a closure that is glued or heat-sealed. Preferably, the container is adapted to attach it to a crop, for example by comprising a hook to hang it from a crop leaf or branch or by comprising a sticky surface to stick it to a surface of the crop.

The present invention further pertains to a method for rearing predatory mites, the method comprising
- providing a composition of the invention, and
- allowing individuals of the predatory mite population to feed on said prey mite population.

Preferably, the individuals of the predatory mite population are allowed to feed on said prey mite population while maintaining said mite composition at 5 to 35 °C and 30 to 100% relative humidity; in particular at 15 to 35°C and 50 to 100% relative humidity.

As described herein before, in another embodiment, the method for rearing predatory mites further comprises adding a fungus. In a particular embodiment, the fungus is a freeze-dried fungus. For example, present invention provides a method for rearing predatory mites, the method comprising:
- generating a mite composition by contacting a carrier with a prey mite population, a predatory mite population, and a culture of fungus, and
- allowing individuals of the predatory mite population to feed on said prey mite population. In principle, the fungus can be added at any time during the rearing method and will then provide benefits, during rearing and/or during use of the resulting composition for biological control. In a particular embodiment, the fungus is added such that the fungus is present while the individuals of the predatory mite population feed on the prey mite population. In a preferred embodiment, the fungus is added before the predatory mite population is allowed to feed on said prey mite population. In a further embodiment, the rearing method of the invention comprises:

- contacting a carrier with a prey mite population and a fungus,
- rearing said prey mite population on said carrier in the presence of said fungus,
- contacting said reared prey mite population with a predatory mite population, and
- allowing individuals of the predatory mite population to feed on said prey mite population.

Beneficially, the carrier can be incubated with a fungus before the prey mite population is added. This allows for the growth of the fungus on the carrier and ensures a sufficient food source for the prey mite population.

Advantageously, when the reared prey mite population is contacted with the predatory mite population, the prey mite population can be retained on the carrier and in the presence of the fungus. There is thus no need to isolate the prey mite population from the carrier and fungus before contacting it with the predatory mite population. In addition, the presence of fungus during rearing of predatory mites provides the benefits as described herein. In particular, the fungus may act as a food source for the prey mite population, such that the prey mite population can develop further while it is preyed upon by the growing predatory mite population.

The present invention further pertains to a use of a mite composition according to the invention to control fungal growth in a predatory mite rearing system, wherein the mites are fungus-feeding mites.

The present invention further pertains to a method for controlling a pest in a crop, the method comprising providing to said crop a mite composition according to the invention. The mite composition of the invention may be distributed directly onto the crop. Alternatively, the mite composition of the invention is provided in the proximity of the crop. For example, the mite composition of the invention may be provided to a crop by placing a container holding the mite composition in the vicinity of the crop and allowing the predatory mites to exit from said container. This way, predatory mites spread themselves through the crops and control the crop pests. In a preferred embodiment, the pest is an arthropod pest.

These and other embodiments of the invention are indicated in the appended claims.

The invention will now be further described with reference to the following examples, which show non-limiting embodiments of different aspects of the invention.

### Examples

### Example 1: Oviposition rate of Amblyseius swirskii Athias-Henriot on different prey mites.

The first example was conducted to evaluate the nutritional quality of a tarsonemid prey mite as compared to two standard used astigmatid prey mites for a phytoseiid predatory mite. More specifically, the oviposition rate of the predatory mite *Amblyseius swirskii* Athias-Henriot (Acari: Mesostigmata: Phytoseiidae) was tested on the tarsonemid prey mite *Tarsonemus fusarii* Cooreman (TF) (Acari: Prostigmata: Tarsonemidae) as compared to two astigmatid prey mites, namely *Carpoglyphus lactis* L. (CL) (Acari: Astigmata: Carpoglyphidae) and *Thyreophagus entomophagus* (Laboulbene) (TE) (Acari: Astigmata: Acaridae).

Prey mites were cultured on a medium containing bran, wheat germ and yeast in plastic containers (8 × 5,7 cm) with a ventilated lid. The containers were maintained at a temperature of 22 ± 1°C and a relative humidity of 85 ± 5%.

A single gravid female *A. swirskii* was transferred from mass-rearing facilities onto a black PVC arena (2 × 4 cm) placed on a layer of wet cotton. The edges of the arena were covered with tissue paper to prevent mites from escaping. A black piece of plastic (1×1 cm) with cotton threads was added to provide shelter and oviposition substrates. Prey mites were provided ad libitum. The number of eggs deposited by each female was counted daily during 4 consecutive days. The oviposition rate of the first day was omitted from analysis to limit the effect of the diet prior to the experiment (Sabelis 1990). Twelve replications were used for each treatment. The sum of the amount of eggs laid during day 2, 3 and 4 were compared with GLM model with Poisson distribution. Contrasts among treatments were determined with general linear hypothesis testing (function glht of the package Ismeans in R, Lenth 2016). All analyses were performed using the statistical software R 3.10 (RDevelopment Core Team 2012).

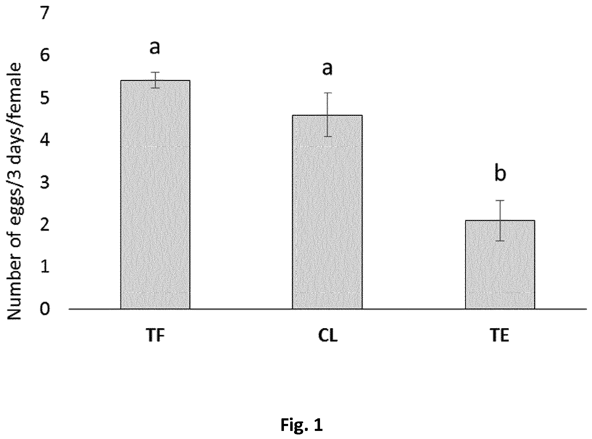

The results demonstrate that *A. swirskii* is able to reproduce on the tarsonemid prey mite *T. fusarii.* Egg-laying was influenced by the prey specie (GLM, χ2 = 20.3, d.f. = 2, p<0.001) with that on *C. lactis* and significantly better than a diet consisting of *T. entomophagus* (Fig. 1). More eggs were laid when grown on the tarsonemid prey mite in comparison to the astigmatid prey mites, although this didn't reach statistical significance in relation to *C. lactis.* In conclusion, tarsonemid mites are a suitable prey mite population for the commercial rearing of predatory mites, such as *A. swirskii.*

### Example 2: Capture success rate of A. swirskii when provided different developmental stages of prey mites

Previous studies have reported that adult stages of astigmatid prey mites are more difficultly subdued and consumed than eggs and larvae of these mites. In the present example, the rate of a capture success rate was assessed for the eggs, larvae and adults of the tarsonemid prey mite *T. fusarii*, and the astigmatid prey mites *C. lactis* and *T. entomophagus.*

Predatory and prey mites were reared as described in example 1. Female *A. swirskii* were starved for 16h prior to start of the experiment by transferring them to a black PVC plate without any food source. After 16h, a single female *A. swirskii* was transferred to a black PVC arena as described in experiment 1. Prior to the introduction of the *A. swirskii* female, either 50 eggs, 50 larvae or 25 adults of the three prey mites were transferred to the experimental arena. After introduction of the predator, observations were done for 5 minutes or until a successful attack (i.e. killing and feeding on the prey). If a female did not succeed in killing prey within 5 minutes, this was recorded as unsuccessful. For each treatment, 15 replicates were set up. Data were compared among treatments with a GLM model with binomial distribution. Contrasts among treatments were assessed by stepwise model simplification through aggregation of nonsignificant factor levels (Crawley 2013). All statistical analyses were done using the computer software R version 3.1.0 (R Core Team 2014).

When tarsonemid *T. fusarii* eggs were provided, all females successfully fed on the eggs within 5 minutes. Surprisingly, less than 30% of the females were capable of killing a *C. lactis* egg and none of the females killed *T. entomophagus* eggs. For the adults, also a higher proportion of prey individuals were subdued when the tarsonemid prey mite *T. fusarii* was provided as compared to the other astigmatid prey mite species. For larvae, all females killed larvae within 5 minutes.

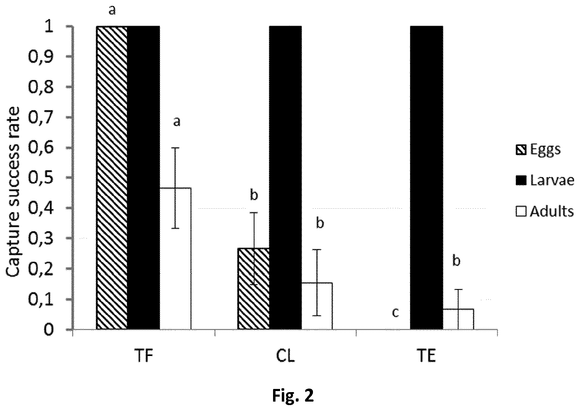

Capture rate was equal between all species when the prey were larvae (GLM, χ2 = 2 ×10-10, d.f. = 2, p=0.05). However, the capture rate was influenced by species when eggs (GLM, χ2 = 1315.3, d.f. = 2, p<0.05) and adults (GLM, χ2 = 39.2, d.f. = 2, p<0.05) were offered. From the results presented in Fig. 2, it is clear that *A. swirskii* females are able to kill and consume much more of the eggs and adults of the tarsonemid prey mite *T. fusarii* as compared to the conventionally used astigmatid prey mite species *C. lactis* and *T. entomophagus.* Thus, for tarsonemid prey mites, all developmental stages are more easily subdued and killed, making it a more suitable prey.

### Example 3: Oviposition rate of Homeopronematus anconai on Tarsonemus fusarii.

This experiment was conducted to evaluate the nutritional quality of a tarsonemid prey mite for a prostigmatid predatory mite, namely *Homeopronematus anconai* (Baker).

Prey mites were cultured on a medium containing bran, wheat germ and yeast in plastic containers (8 × 5,7 cm) with a ventilated lid. The containers were maintained at a temperature of 22 ± 1°C and a relative humidity of 85 ± 5%.

A single gravid female *H. anconai* was transferred from mass-rearing facilities onto a tomato leaf arena (2 × 4 cm) placed on a layer of wet cotton. The edges of the arena were covered with tissue paper to prevent mites from escaping. Prey mites were provided ad libitum.

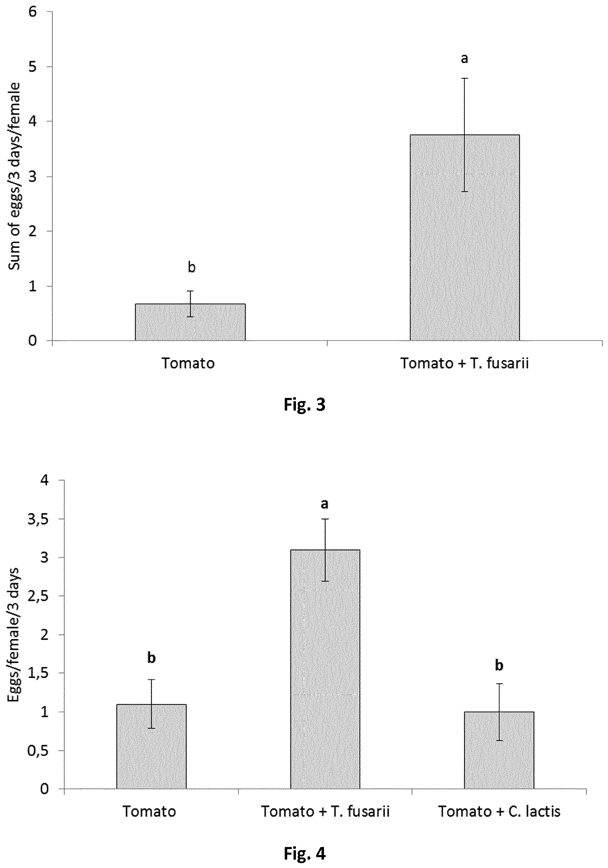

The number of eggs deposited by each female was counted daily during 4 consecutive days. The oviposition rate of the first day was omitted from analysis to limit the effect of the diet prior to the experiment (Sabelis 1990). Ten replications were used for each treatment. The sum of the amount of eggs laid during day 2, 3 and 4 were compared with GLM model with Poisson distribution using the statistical software R 3.10 (R Development Core Team 2012). Results are shown in Fig. 3. Providing *T. fusarii* on the tomato leaf arena resulted in a significantly higher number of eggs laid by *H. anconai* (GLM, χ2 = 20.418, d.f. = 1, p<0.001), supporting that tarsonemids can be used as prey mites for rearing prostigmatid predatory mites.

### Example 4: Oviposition rate of Pronematus ubiauitus on Tarsonemus fusarii and Carpoglyphus lactis.

This experiment was conducted to evaluate the nutritional quality of a tarsonemid prey mite for another prostigmatid predatory mite, namely *Pronematus ubiquitus* (Baker).

Prey mites were cultured on a medium containing bran, wheat germ and yeast in plastic containers (8 × 5,7 cm) with a ventilated lid. The containers were maintained at a temperature of 22 ± 1°C and a relative humidity of 85 ± 5%.

A single gravid female *P. ubiquitus* was transferred from mass-rearing facilities onto a tomato leaf arena (2 × 4 cm) placed on a layer of wet cotton. The edges of the arena were covered with tissue paper to prevent mites from escaping. Prey mites were provided ad libitum.

The number of eggs deposited by each female was counted daily during 4 consecutive days. The oviposition rate of the first day was omitted from analysis to limit the effect of the diet prior to the experiment (Sabelis 1990). Ten replications were used for each treatment. The sum of the amount of eggs laid during day 2, 3 and 4 were compared with GLM model with a quasipoisson distribution to correct for overdispersion using the statistical software R 3.10 (R Development Core Team 2012). Results are shown in Fig. 4.

A significantly higher oviposition rate of *P. ubiquitus* females was observed when fed with *T. fusarii* as compared to *C. lactis* and a control treatment without food (GLM, χ2 = 15.039, d.f. = 2, p=0.002).

Tarsonemid prey mites thus have potential to be used in the rearing of prostigmatid predatory mites, such as *P. ubiquitus,* whereas traditionally used feed mites, such as *C. lactis,* do not.

### Example 5: Comparison of the oviposition rate of Amblvseius swirskii when fed Tarsonemus confusus and Tarsonemus fusarii.

As shown in examples 1 and 2, *A. swirskii* can be reared successfully on *Tarsonemus fusarii.* In the current example, the oviposition rate of *Amblyseius swirskii* on *Tarsonemus confusus* was tested in comparison to *Tarsonemus fusarii.*

Both tarsonemid mites were cultured on a medium containing bran, wheat germ and yeast in plastic containers (8 × 5,7 cm) with a ventilated lid. The containers were maintained at a temperature of 22 ± 1°C and a relative humidity of 85 ± 5%.

A single gravid female *A. swirskii* was transferred from mass-rearing facilities onto a black PVC arena (2 × 4 cm) placed on a layer of wet cotton. The edges of the arena were covered with tissue paper to prevent mites from escaping. A black piece of plastic (1×1 cm) with cotton threads was added to provide shelter and oviposition substrates. Prey mites were provided ad libitum. The number of eggs deposited by each female was counted daily during 3 consecutive days. Per treatment, 12 replicates were set up. The oviposition rate of the first day was omitted from analysis to limit the effect of the diet prior to the experiment (Sabelis 1990). The sum of the amount of eggs laid during day 2 and 3 was compared using GLM analysis (with quasipoisson distribution). Contrasts among treatments were determined with general linear hypothesis testing (function glht of the package Ismeans in R, Lenth 2016). Analysis was performed using the statistical software R 3.10 (R Development Core Team 2012).

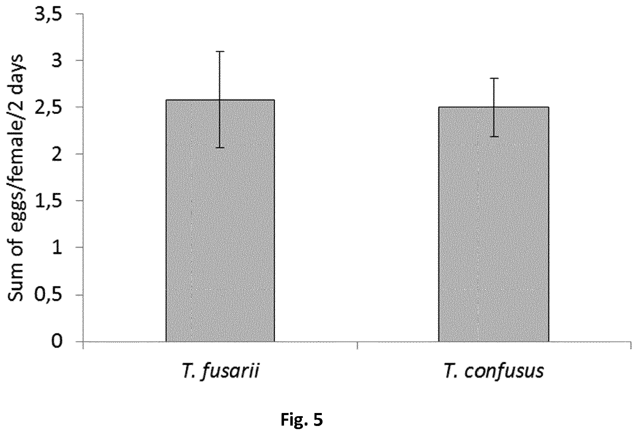

The results of the experiment are shown in Fig. 5. A similar number of eggs was laid by *A. swirskii* when offered either *T. fusarii* or *T. confusus* (GLM; χ²=0.0164, df=1, p=0.898). This result confirms that other tarsonemid species from the genus *Tarsonemus*, such as *T. confusus*, can be used as a food source for mass-production of predatory mites.

### Example 6: Oviposition rate of Amblyseius swirskii on Fungitarsonemus sp.

This experiment was conducted to evaluate the nutritional quality of another tarsonemid prey mite, namely a *Fungitarsonemus* sp.

*Fungitarsonemus* sp. mobiles and eggs were collected from ivy leaves (*Hedera helix* L.). Prey mites were transferred to ivy leaf discs (2 × 4 cm) placed upside down on a layer of wet cotton. The edges of the leaf disc were covered with moist tissue paper to prevent the mite from escaping. A single gravid female *A. swirskii,* starved for 24h prior to the experiment, was transferred to the leaf disc. Fresh *Fungitarsonemus* mobiles and eggs were transferred to the experimental leaf arena daily, in order to provide prey mites *ad libitum.* A black piece of plastic (1×1 cm) with cotton threads was added to provide shelter and oviposition substrates. The number of eggs deposited by the females was counted daily for two consecutive days. The leaf discs were kept in a climatic chamber set at a temperature of 22 ± 1°C and a relative humidity of 85 ± 5%. Per treatment, 6 replicates were set up. The sum of the eggs laid during the two days was compared using GLM analysis (with poisson distribution). Contrasts among treatments were determined with general linear hypothesis testing (function glht of the package Ismeans in R, Lenth 2016). Analysis was performed using the statistical software R 3.10 (R Development Core Team 2012).

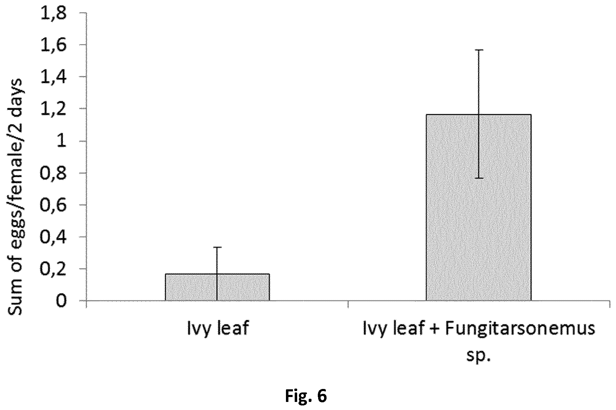

The results are shown in Fig. 6. A significant higher amount of eggs was counted when the *Fungitarsonemus* sp. was offered on the ivy leaf discs (GLM, χ²=5.062, df=1, p=0.0245). This confirms that tarsonemids outside of the *Tarsonemus* genus can be used for predatory mite rearing as well.

## Claims

1. A mite composition comprising:
- a predatory mite population,
- a prey mite population, and
- a carrier for individuals of said populations,
wherein said prey mite population comprises fungus-feeding mites from the family of the Tarsonemidae,
wherein said mite composition comprises at least 1,000 mites of the prey mite population per gram of the composition, and
wherein said carrier is a solid material which is suitable to provide a carrier surface to the individuals of both the predatory and the prey mite populations,
wherein said mite composition does not comprise a living mite species selected from the group consisting of *Polyphagotarsonemus latus* and *Phytonemus pallidus.*

2. The composition according to claim 1, wherein said prey mite population comprises at least one mite species selected from the following genera:
- the genus Tarsonemus, e.g. *Tarsonemus granarius, Tarsonemus floricolus*, *Tarsonemus myceliophagus*, *Tarsonemus subcorticalis*, *Tarsonemus minimax; Tarsonemus fusarii*;
- the genus Fungitarsonemus, e.g. *Fungitarsonemus peregrinus*, *Fungitarsonemus pulvirosus*
- the genus Heterotarsonemus, e.g. *Heterotarsonemus lindquisti*, *Heterotarsonemus coleopterorum*;
- the genus Daidalotarsonemus, e.g. *Daidalotarsonemus vandevriei, Diadalotarsonemus tesselatus*;
- the genus Neotarsonemoides, e.g. *Neotarsonemoides denigratus*
- the genus Pseudacarapis, e.g. *Pseudacarapis indoapis*;

3. The composition according to claim 1 or 2, further comprising a food source for said mite populations.

4. The composition of any one of the previous claims, wherein the food source for said prey mite population contains non-photosynthetic plant material, such as wheat germ or pollen.

5. The composition of any one of the previous claims, wherein said carrier comprises grains of a grass species or any part thereof, such as germ or bran.

6. The composition of any one of the previous claims, wherein said carrier comprises carrier elements with an average longest axis of between 1.0 and 20.0 mm.

7. The composition of any one of previous claims, wherein the number of individuals of the predatory mite species relative to the number of individuals of the prey mite is from about 1:1 to about 1:500; in particular from about 1:50 to about 1:200; more in particular between 1:75 and 1:125.

8. The composition of any one of previous claims, wherein said predatory mite species are mesostigmatid or prostigmatid mite species, in particular Phytoseiidae.

9. The composition of any one of the previous claims, further comprising a fungus from the Hypocreacea.

10. A container comprising the composition of any one of the previous claims.

11. A method for rearing predatory mites, the method comprising
- providing the composition of any one of claims 1 to 9, and
- allowing individuals of the predatory mite population to feed on said prey mite population.

12. The method of claim 11, further comprising maintaining said mite composition at 5 to 35 °C and 30 to 100% relative humidity.

13. Use of the mite composition of claim 1 to control fungal growth in a predatory mite rearing system, wherein the mites are fungus-feeding mites.

14. A method for controlling a pest in a crop, the method comprising providing to said crop a mite composition according to any one of claims 1 to 9.

## Patentansprüche

1. Eine Milbenzusammensetzung, welche Folgendes umfasst:
- eine Raubmilbenpopulation,
- eine Beutemilbenpopulation, und
- einen Träger für Individuen der Populationen,
wobei die Beutemilbenpopulation pilzfressende Milben aus der Familie der Tarsonemidae umfasst,
wobei die Milbenzusammensetzung mindestens 1.000 Milben der Beutemilbenpopulation pro Gramm der Zusammensetzung umfasst, und wobei der Träger ein festes Material ist, welches geeignet ist, um für die Individuen sowohl der Raub- als auch der Beutemilbenpopulation eine Trägeroberfläche bereitzustellen,
wobei die erwähnte Milbenzusammensetzung keine lebende Milbenart umfasst, die aus der Gruppe bestehend aus *Polyphagotarsonemus latus* und *Phytonemus pallidus* ausgewählt ist.

2. Die Zusammensetzung nach Anspruch 1, wobei die Beutemilbenpopulation zumindest eine aus den folgenden Gattungen ausgewählte Milbenart umfasst:
- die Gattung Tarsonemus, z. B. *Tarsonemus granarius*, *Tarsonemus floricolus*, *Tarsonemus myceliophagus*, *Tarsonemus subcorticalis*, *Tarsonemus minimax*; *Tarsonemus fusarii*;
- die Gattung Fungitarsonemus, z. B. *Fungitarsonemus peregrinus*, *Fungitarsonemus pulvirosus*
- die Gattung Heterotarsonemus, z. B. *Heterotarsonemus lindquisti*, *Heterotarsonemus coleopterorum*;
- die Gattung Daidalotarsonemus, z. B. *Daidalotarsonemus vandevriei*, *Daidalotarsonemus tesselatus*;
- die Gattung Neotarsonemoides, z. B. *Neotarsonemoides denigratus*
- die Gattung Pseudacarapis, z. B. *Pseudacarapis indoapis*;

3. Die Zusammensetzung nach Anspruch 1 oder 2, welche ferner eine Nahrungsquelle für die Milbenpopulationen umfasst.

4. Die Zusammensetzung nach irgendeinem der vorigen Ansprüche, wobei die Nahrungsquelle für die Beutemilbenpopulation nicht-fotosynthetisches Pflanzenmaterial wie Weizenkeim oder Pollen umfasst.

5. Die Zusammensetzung nach irgendeinem der vorigen Ansprüche, wobei der Träger Körner einer Grasart oder irgendeinen Teil davon wie Keim oder Kleie umfasst.

6. Die Zusammensetzung nach irgendeinem der vorigen Ansprüche, wobei der Träger Trägerelemente mit einer durchschnittlich längsten Achse von zwischen 1,0 und 20,0 mm umfasst.

7. Die Zusammensetzung nach irgendeinem der vorigen Ansprüche, wobei die Anzahl von Individuen der Raubmilbenart im Verhältnis zur Anzahl von Individuen der Beutemilbe von etwa 1:1 bis etwa 1:500 ist, insbesondere von etwa 1:50 bis etwa 1:200; genauer zwischen 1:75 und 1:125.

8. Die Zusammensetzung nach irgendeinem der vorigen Ansprüche, wobei die Raubmilbenarten mesostigmatide oder prostigmatide Milbenarten sind, insbesondere Phytoseiidae.

9. Die Zusammensetzung nach irgendeinem der vorigen Ansprüche, welche ferner einen Pilz der Hypocreaceae umfasst.

10. Ein Behälter, welcher die Zusammensetzung nach irgendeinem der vorigen Ansprüche umfasst.

11. Ein Verfahren zur Aufzucht von Raubmilben, wobei das Verfahren Folgendes umfasst:
- Bereitstellen der Zusammensetzung nach irgendeinem der Ansprüche 1 bis 9, und
- Zulassen, dass sich Individuen der Raubmilbenpopulation von der Beutemilbenpopulation ernähren.

12. Das Verfahren nach Anspruch 11, welches ferner das Halten der Milbenzusammensetzung auf 5 bis 35 °C und 30 bis 100 % relativer Feuchtigkeit umfasst.

13. Verwendung der Milbenzusammensetzung nach Anspruch 1 zur Bekämpfung des Pilzwachstums in einem Raubmilbenaufzuchtsystem, wobei die Milben pilzfressende Milben sind.

14. Ein Verfahren zur Bekämpfung eines Schädlings in einer Nutzpflanze, wobei das Verfahren das Bereitstellen einer Milbenzusammensetzung nach irgendeinem der Ansprüche 1 bis 9 für die Nutzpflanze umfasst.

## Revendications

1. Composition à base d'acariens comprenant :
- une population d'acariens prédateurs,
- une population d'acariens proies, et
- un support pour les individus desdites populations,
dans laquelle ladite population d'acariens proies comprend des acariens fongivores de la famille des Tarsonemidae,
dans laquelle ladite composition à base d'acariens comprend au moins 1 000 acariens de la population d'acariens proies par gramme de composition, et
dans laquelle ledit support est un matériau solide qui convient pour fournir une surface de support aux individus des populations d'acariens prédateurs et proies,
dans laquelle ladite composition à base d'acariens ne comprend pas d'espèces d'acariens vivants choisies dans le groupe constitué de *Polyphagotarsonemus latus* et *Phytonemus pallidus.*

2. Composition selon la revendication 1, dans laquelle ladite population d'acariens proies comprend au moins une espèce d'acariens sélectionnée parmi les genres suivants :
- le genre Tarsonemus, par exemple *Tarsonemus granarius, Tarsonemus floricolus, Tarsonemus myceliophagus, Tarsonemus subcorticalis*, *Tarsonemus minimax; Tarsonemus fusarii ;*
- le genre Funaitarsonemus, par exemple *Fungitarsonemus peregrinus*, *Fungitarsonemus pulvirosus*
- le genre Heterotarsonemus, par exemple *Heterotarsonemus lindquisti*, *Heterotarsonemus coleopterorum ;*
- le genre Daidalotarsonemus, par exemple *Daidalotarsonemus vandevriei*, *Diadalotarsonemus tesselatus;*
- le genre Neotarsonemoides, par exemple *Neotarsonemoides denigratus*
- le genre Pseudacarapis, par exemple *Pseudacarapis indoapis*;

3. Composition selon la revendication 1 ou 2, comprenant en outre une source de nourriture pour lesdites populations d'acariens.

4. Composition de l'une quelconque des revendications précédentes, dans laquelle la source de nourriture de ladite population d'acariens proies contient des matières végétales non photosynthétiques, telles que le germe de blé ou le pollen.

5. Composition de l'une quelconque des revendications précédentes, dans laquelle ledit support comprend des grains d'une espèce d'herbes ou une partie de ceux-ci, tels que le germe ou le son.

6. Composition de l'une quelconque des revendications précédentes, dans laquelle ledit support comprend des éléments de support dont l'axe le plus long est compris entre 1,0 et 20,0 mm.

7. Composition de l'une quelconque des revendications précédentes, dans laquelle le nombre d'individus des espèces d'acarien prédateur par rapport au nombre d'individus de l'acarien proie est d'environ 1:1 à environ 1:500 ; en particulier d'environ 1:50 à environ 1:200 ; plus particulièrement entre 1:75 et 1:125.

8. Composition selon l'une quelconque des revendications précédentes, dans laquelle les espèces d'acariens prédateurs sont des espèces d'acariens mésostigmatidés ou prostigmatidés, en particulier des Phytoseiidae.

9. Composition de l'une quelconque des revendications précédentes, comprenant en outre un champignon de la famille des Hypocréacées.

10. Récipient comprenant la composition de l'une quelconque des revendications précédentes.

11. Méthode pour élever des acariens prédateurs, comprenant les étapes consistant à
- fournir la composition de l'une quelconque des revendications 1 à 9, et
- permettre aux individus de la population d'acariens prédateurs de se nourrir de ladite population d'acariens proies.

12. Méthode de la revendication 11, comprenant en outre le maintien de ladite composition à base d'acariens de 5 à 35 °C et à une humidité relative de 30 à 100 %.

13. Utilisation de la composition à base d'acariens de la revendication 1 pour contrôler la croissance fongique dans un système d'élevage d'acariens prédateurs, dans lequel les acariens sont des acariens fongivores.

14. Méthode de lutte contre un parasite dans une culture, consistant à fournir à ladite culture,une composition à base d'acariens selon l'une quelconque des revendications 1 à 9.
